(19) Europäisches Patentamt
European Patent Office
Office européen des brevets

(11) Publication number: 0 032 708
B1

(12) # EUROPEAN PATENT SPECIFICATION

(45) Date of publication of patent specification: 04.05.83

(51) Int. Cl.³: C 07 D 209/20, C 07 D 209/24, A 61 K 31/405

(21) Application number: 81100186.6

(22) Date of filing: 13.01.81

(54) **Tryptophan derivative, process for preparing same and a pharmaceutical composition containing such derivative.**

(30) Priority: 18.01.80 JP 5196/80

(43) Date of publication of application:
29.07.81 Bulletin 81/30

(45) Publication of the grant of the patent:
04.05.83 Bulletin 83/18

(84) Designated Contracting States:
DE FR GB IT

(56) References cited:
US - A - 4 000 297
US - A - 4 001 276

(73) Proprietor: Tanabe Seiyaku Co., Ltd.
No. 21 Dosho-machi 3-chome Higashi-ku
Osaka-shi Osaka-fu (JP)

(72) Inventor: Takashima, Kohki
No. 32, Kasumigaoka
Shinjuku-ku Tokyo-to (JP)
Inventor: Shigezane, Keisuke
No. 640-16, Oaza-Nishibori
Urawa-shi Saitama-ken (JP)
Inventor: Saiga, Yutaka
No. 699-14, Oaza-Ageo-Shimo
Ageo-shi Saitama-ken (JP)

(74) Representative: Hansen, Bernd, Dr.rer.nat. et al,
Hoffmann . Eitle & Partner Patentanwälte
Arabellastrasse 4
D-8000 München 81 (DE)

EP 0 032 708 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England

Tryptophan derivative, process for preparing same and a pharmaceutical composition containing such derivative

This invention relates to novel tryptophan derivatives, processes for preparing same and a pharmaceutical composition containing such derivatives. More particularly, it relates to N α-benzylthiocarbonyl-tryptophan derivatives of the formula:

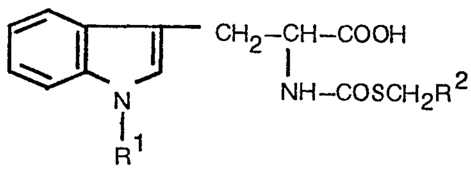

(I)

wherein $R^1$ is hydrogen or lower alkyl and $R^2$ is phenyl or lower alkyl-phenyl, or a pharmaceutically acceptable salt thereof.

Hyperlipidemia is known to be one of important causative factors of arteriosclerosis, and various compounds such as dextran sulfate, simfibrate (chemical name: 2-(4-chlorophenoxy)-2-methylpropanoic acid 1,3-propanediyl ester), nicomol (chemical name: nicotonic acid, 1,1,3,3-tetraester with 2-hydroxy-1,1,3,3-cyclohexanetetramethanol), clofibrate (chemical name: 2-(4-chlorophenoxy)-2-methylpropanoic acid ethyl ester) and vitamin E nicotinate have been used for the treatment or prophylaxis of said hyperlipidemia.

As a result of investigations, it has been found that the tryptophan derivative (I) of the present invention is useful as hypolipidemic agent. In particular, the tryptophan derivative (I) of the invention shows potent hypolipidemic activity without undesirable side effects such as hepatic disfunction.

Representative examples of said tryptophan derivative include those of the formula (I) in which $R^1$ is hydrogen or lower alkyl such as methyl, ethyl and propyl; and $R^2$ is phenyl or lower alkyl-phenyl such as 4-methylphenyl, 4-ethylphenyl and 4-isopropylphenyl. Among them, a preferred group of compounds are those of the formula (I) in which $R^1$ is hydrogen or methyl; and $R^2$ is phenyl or 4-methylphenyl.

According to the present invention, the tryptophan derivative (I) is prepared by reacting a tryptophan compound of the formula:

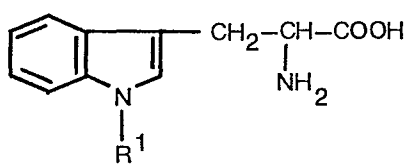

(II)

wherein $R^1$ is the same as defined above, with carbonyl sulfide (COS) and a benzyl halide of the formula:

$$R^2CH_2X \qquad (III)$$

wherein X is halogen and $R^2$ is the same as defined above. Alternatively, the tryptophan derivative (I) may be prepared by reacting the tryptophan compound (II) with a benzylthiocarbonyl halide of the formula:

$$R^2CH_2SCOX \qquad (IV)$$

wherein $R^2$ and X are the same as defined above.

The reaction of the compound (II) with carbonyl sulfide and the compound (III) as well as the reaction of the compound (II) with the compound (IV) are accomplished in the presence of an acid acceptor in a solvent. Suitable examples of said acid acceptor include inorganic bases such as sodium hydroxide, sodium carbonate, sodium bicarbonate and ammonia; and organic bases such as triethylamine, N-methylmorpholine, N,N-dimethylaniline, pyridine and anionic ion exchange resins. Water or a mixture of water and an organic solvent such as tetrahydrofuran, dioxane, acetonitrile, dimethylsulfoxide, N,N'-dimethylformamide, ether, ethyl acetate, methylene chloride or chloroform are suitable as the solvent. The reaction of the compound (II) with carbonyl sulfide and the compound (III) is preferably carried out at a temperature between −30°C and 50°C, especially at a temperature between −10°C and 25°C. On the other hand, the reaction of the compounds (II) and (IV) is preferably carried out at a temperature between −50°C and 50°C, especially at a temperature between −20°C and 25°C.

In view of the potent hypolipidemic activity of the tryptophan derivative (I) obtained above, said derivative of the present invention is useful for the therapeutic treatment or prophylaxis of various hyperlipidemias such as hypercholesterolemia, hypertriglyceridemia, hyperlipemia and the like. Moreover, the tryptophan derivative (I) of the invention shows less hepatomegaly or other hepatic

disorders as compared with clofibrate, and is particularly suitable for treatment of the above-mentioned diseases without such undesirable side effects. Further, the tryptophan derivative (I) has a potent preventive effect upon lipid peroxide formation in living bodies. Lipid peroxide levels in tissues of mammalian species are known to increase with age and cause cell death and/or damage with a consequent change of cell permeability. In addition, lipid peroxides have been suggested to be a primary etiologic factor in the genesis of stroke (cf., "Stroke" Vol. 10, No. 3, pages 323—326 (1979)). Thus, the tryptophan derivative (I) may be used to improve the lipid peroxide levels in the tissues of aged subjects.

The tryptophan derivatives (I) can be used for pharmaceutical use in the form of either the free acid or a pharmaceutically acceptable salt thereof. Examples of the pharmaceutically acceptable salts include alkali metal salts such as sodium, potassium or lithium salts; alkali earth metal salts such as calcium or magnesium salts; salts with non-toxic amines such as methylamine, ethylamine, diethylamine, triethylamine, benzylamine, pyrrolidine, piperidine, cyclohexylamine, dicylohexylamine, ethanolamine or diethanolamine; salts with basic amino acids such as ornithine, lysine or arginine; salt with carnitine; and the like.

The tryptophan derivative (I) and a pharmaceutically acceptable salt thereof may be administered either orally or parenterally. For oral administration, said derivative or a salt thereof may be used in the form of tablets, coated tablets, powder, capsules, granules and the like. Known medicinal excipients such as calcium carbonate, calcium phosphate, corn starch, potato starch, sugar, lactose, talc, magnesium stearate and so forth may be used in making these pharmaceutical preparations. Alternatively, said tryptophan derivative or a salt thereof may be used for oral administration in the form of aqueous or oily suspensions, solutions, syrups or elixirs. On the other hand, injections and suppositories are suitable for parenteral administration of the tryptophan derivative (I) or its salts, and said injections may be made in the form of solutions or suspensions, if required, in conjunction or admixture with distilled water, essential oils (e.g., peanut oil, corn oil) or non-aqueous solvents (e.g., polyethyleneglycol, polypropyleneglycol, lanoline, coconut oil). Concomitantly, while the present invention includes within its scope of any one of the DL-, D- and L-isomers of the tryptophan derivative (I), L-isomer of said derivative is particularly suitable for pharmaceutical use because of its potent therapeutic effects and less side effects.

Practical and presently preferred embodiments of the present invention are illustratively shown in the following lines. Throughout the specification, each compounds of the present invention have been named according to the "NOMENCLATURE OF $\alpha$-AMINO ACIDS" described in "Biochemistry" *14* (No. 2), 449—462 (1975).

Experiments

(Methods)

(i) Hypolipidemic activity:

A test compound (0.10 w/w %) was added to a commercial diet, and male SD rats (body weight: 120—140 g, a group of 6 rats) were maintained on the diet for one week. After the experimental period, blood was collected from the tail vein of the rats under ether anesthesia. Thereafter, the liver was excised and weighed. Serum cholesterol and serum triglyceride were measured according to the methods of Zak (Amer. J. Clin. Pathol., Vol. 24, page 1307 (1954)) and Van Handel Zilversmit (J. Lab. & Clin. Med., Vol. 50, page 152 (1957)), respectively. Then, the increase (%) in liver weight and the decrease (%) in serum cholesterol or triglyceride were calculated according to the following formulae:

$$\text{Decrease (\%) in serum cholesterol or triglyceride} = \left[1 - \frac{\text{Serum cholesterol or triglyceride (mg/ml) in the medicated group}}{\text{Serum cholesterol or triglyceride (mg/ml) in the control group}}\right] \times 100$$

$$\text{Increase (\%) in liver weight} = \left[\frac{\text{Liver weight in the medicated group}}{\text{Liver weight in the control group}} - 1\right] \times 100$$

(ii) Preventive effect on lipid peroxide formation.

An ethanol solution containing $10^{-3}$ or $10^{-4}$ M of a test compound was added to a mixture of 2.4

ml of 0.067 M potassium phosphate buffer solution (pH 7.4) and 0.5 ml of 10% rat brain homogenate to adjust the final volume of the mixture to 3 ml. After 30-minute incubation of the mixture at 37°C, one ml of 20% trichloroacetic acid was added thereto, and lipid peroxide formation was determined by the thiobarbituric acid colorimetric method (J. Robak et al., Viochem. Pharmacol., Vol. 25, page 2233 (1976)). Suppression (%) of lipid peroxide formation of the test compound was calculated according to the following formula:

$$\text{Suppression (\%) of lipid peroxide formation} = \left[1 - \frac{\Delta\ \text{OD of test group*}}{\Delta\ \text{OD of control group**}}\right] \times 100$$

Note: *: group containing the test compound
**: group containing an equal volume of ethanol instead of the test compound solution
ΔOD was calcualted as [(optical density measured at 532 nm) — (optical density measured at 600 nm)]

(Results)

The results are shown in the following Table

TABLE

| Test compounds | Decrease (%) in serum trigly-ceride | Decrease (%) in serume choles-terol | Increase (%) in liver weight | Suppression (%) in lipid peroxide formation Dose $10^{-3}$M, | $10^{-4}$M |
|---|---|---|---|---|---|
| (The compounds of the present invention) | | | | | |
| $N^{\alpha}$-(4-methylbenzylthio-carbonyl)-L-tryptophan | 48 | 14 | 0 | 72.7 | 10.2 |
| $N^1$-methyl-$N^{\alpha}$-(benzyl-thiocarbonyl)-L-tryptophan | 30 | 15 | 5 | 65.2 | 9.0 |
| (Control) Clofibrate | 34 | 26 | 14 | 24.3 | 0.5 |

Example 1

10.2 g of L-tryptophan and 10.1 g of triethylamine are dissolved in 50 ml of water, and 78 ml of a dioxane solution containing 3.0 g of carbonyl sulfide are added dropwise thereto at 5°C to 10°C for 15 minutes. The mixture is stirred at 10°C for 15 minutes. Then, 7.0 g of 4-methylbenzyl chloride are added dropwise to the mixture at 10°C for 10 minutes, and said mixture is further stirred at room temperature for one hour. After the reaction, the mixture is condensed under reduced pressure to remove solvent. After the residue obtained is dissolved in a mixture of water and ether, the ether layer is removed therefrom, and the aqueous layer is acidified with 10% hydrochloric acid and then extracted with ethyl acetate. The extract is washed with water, dried and evaporated under reduced pressure to remove solvent. The residue thus obtained is crystallized with n-hexane, whereby 15.9 g of $N^{\alpha}$-(4-methylbenzylthiocarbonyl)-L-tryptophan are obtained as pale yellow powder. Yield: 86%

M.p. 111—115°C

$[\alpha]_D^{30}$ + 6.0° (C=1.0, methanol)

Elemental analysis calculated for $C_{20}H_{20}N_2O_3S\cdot1/2H_2O$

| | | | | |
|---|---|---|---|---|
| | C, 63.64; | H, 5.61; | N, 7.42; | S, 8.49 |
| Found | C, 64.18; | H, 5.73; | N, 7.78; | S, 8.15 |

Example 2

5.5 g of $N^1$-methyl-L-tryptophan and 5.1 g of triethylamine are dissolved in 25 ml of water, and 34 ml of a dioxane solution containing 1.5 g of carbonyl sulfide are added dropwise thereto at 5°C to 10°C for 15 minutes. The mixture is stirred at 10°C for 15 minutes. Then, 3.2 g of benzyl chloride are added dropwise to the mixture at 10°C for 10 minutes, and said mixture is further stirred at room

temperature for 2 hours. After the reaction, the mixture is treated in the same manner as described in Example 1. 3.4 g of $N^1$-methyl-$N^{\alpha}$-benzylthiocarbonyl-L-tryptophan are thereby obtained as pale yellow powder. Yield: 37% M.p. 85—87°C.

$[\alpha]_D^{30}$ + 15.0° (C=1.0, methanol)
$IR\nu_{max}^{nujol}$ ($cm^{-1}$): 3300, 1720, 1670, 1500
Mass m/e: 368 ($M^+$)
NMR ($CDCl_3$)$\delta$: 3.27 (d, 2H, J=6.0Hz), 3.56 (s, 3H), 4.10 (s, 2H), 4.67 — 4.97 (m, 1H), 6.05 (d, 1H, J=6.6Hz), 6.74 (s, 1H), 6.92 — 7.56 (m, 4H), 7.22 (s, 5H), 8.12 (s, 1H)

## Claims

1. A tryptophan derivative of the formula

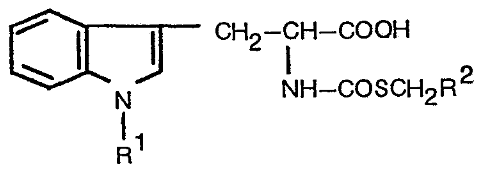

(I)

wherein $R^1$ is hydrogen, methyl, ethyl or propyl, and $R^2$ is phenyl, 4-methyl phenyl, 4-ethyl phenyl or 4-isopropyl phenyl, or a pharmaceutical acceptable salt thereof.

2. The compound of claim 1, in which $R^1$ is hydrogen or methyl.

3. The compound of claim 1, in which $R^2$ is phenyl or 4-methyl phenyl.

4. The compound of claim 1, in which $R^1$ is hydrogen or methyl, and $R^2$ is phenyl or 4-methyl phenyl.

5. The compound of claim 4, in which $R^1$ is hydrogen and $R^2$ is 4-methyl phenyl.

6. The compound of claim 4, in which $R^1$ is methyl and $R^2$ is phenyl.

7. A process for preparing the compounds according to claims 1 to 6, which comprises the steps of:

(A) reacting a compound of the formula:

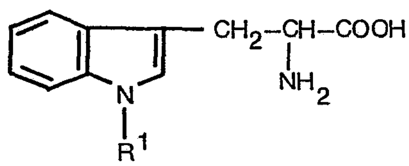

(II)

wherein $R^1$ is the same as defined above, with carbonyl sulfide (COS) and a benzyl halide of the formula:

$$R^2CH_2X \quad (III)$$

wherein X is halogen and $R^2$ is the same as defined above, or
(B) reacting the compound (II) with a compound of the formula:

$$R^2CH_2SCOX \quad (IV)$$

wherein $R^2$ and X are the same as defined above, and
(C) if required, further converting the product into a pharmaceutically acceptable salt thereof.

8. The process according to Claim 7, wherein the reaction of the compound (II) with carbonyl sulfide and the compound (III) is carried out at a temperature between −30°C and 50°C in the presence of an acid acceptor in a solvent, or the reaction of the compound (II) with the compound (IV) is carried out at a temperature between −50°C and 50°C in the presence of an acid acceptor in a solvent.

9. A pharmaceutical composition which comprises a compound according to claims 1—6 and a pharmaceutical acceptable carrier therefore.

## Patentansprüche

1. Tryptophan-Derivat der Formel

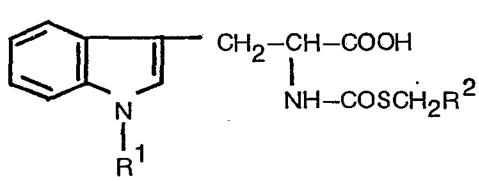

(I)

worin bedeuten:

$R^1$ Wasserstoff, Methyl, Ethyl oder Propyl, und

$R^2$ Phenyl, 4-Methyl-phenyl, 4-Ethyl-phenyl oder 4-Isopropylphenyl, oder

ein pharmazeutisch annehmbares Salz desselben.

2. Verbindung gemäss Anspruch 1, dadurch gekennzeichnet, dass $R^1$ Wasserstoff oder Methyl darstellt.

3. Verbindung gemäss Anspruch 1, dadurch gekennzeichnet, dass $R^2$ Phenyl oder 4-Methyl-phenyl darstellt.

4. Verbindung gemäss Anspruch 1, dadurch gekennzeichnet, dass $R^1$ Wasserstoff oder Methyl und $R^2$ Phenyl oder 4-Methyl-phenyl darstellen.

5. Verbindung gemäss Anspruch 4, dadurch gekennzeichnet, dass $R^1$ Wasserstoff und $R^2$ 4-Methylphenyl darstellen.

6. Verbindung gemäss Anspruch 4, dadurch gekennzeichnet, dass $R^1$ Methyl und $R^2$ Phenyl darstellen.

7. Verfahren zur Herstellung der Verbindungen gemäss den Ansprüchen 1 bis 6, gekennzeichnet durch die folgenden Schritte:

(A) Umsetzung einer Verbindung der Formel:

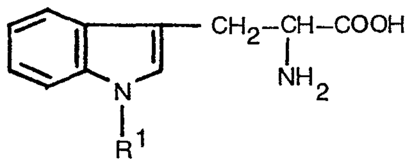

(II)

worin $R^1$ die vorstehend angegebene Bedeutung hat, mit Carbonylsulfid (COS) und einem Benzylhalid der Formel:

$$R^2CH_2X \qquad (III)$$

worin X Halogen bedeutet und $R^2$ die vorstehend angegebene Bedeutung hat, oder

(B) Umsetzung der Verbindung (II) mit einer Verbindung der Formel:

$$R^2CH_2SCOX \qquad (IV)$$

worin $R^2$ und X die vorstehend angegebenen Bedeutungen haben, und

(C) sofern dies erforderlich ist, eine weitere Umwandlung des Produktes in ein pharmazeutisch annehmbares Salz desselben.

8. Verfahren gemäss Anspruch 7, dadurch gekennzeichnet, dass die Reaktion der Verbindung (II) mit Carbonylsulfid und der Verbindung (III) bei einer Temperatur zwischen –30°C und 50°C in Anwesenheit eines Säureakzeptors in einem Lösungsmittel durchgeführt wird, oder die Reaktion der Verbindung (II) mit der Verbindung (IV) bei einer Temperatur zwischen –50°C und 50°C in Gegenwart eines Säureakzeptors in einem Lösungsmittel durchgeführt wird.

9. Pharmazeutisches Mittel, dadurch gekennzeichnet, dass es eine Verbindung gemäss den Ansprüchen 1 bis 6 und einen pharmazeutisch annehmbaren Träger derselben umfasst.

**Revendications**

1. Dérivé du tryptophane de formule:

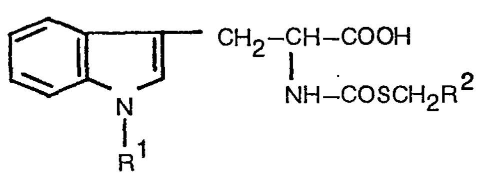

(I)

où $R^1$ est l'hydrogène, un reste méthyle, éthyle ou propyle, et $R^2$ est un reste phényle, 4-méthylphényle, 4-éthylphényle ou 4-isopropylphényle, ou un sel pharmaceutiquement acceptable de ce dérivé.

2. Composé selon la revendication 1, dans lequel $R^1$ est de l'hydrogène ou le reste méthyle.

3. Composé selon la revendication 1, dans lequel $R^2$ est le reste phényle ou 4-méthylphényle.

4. Composé selon la revendication 1, dans lequel $R^1$ est l'hydrogène ou le reste méthyle, et $R^2$ est le reste phényle ou 4-méthylphényle.

5. Composé selon la revendication 4, dans lequel $R^1$ est l'hydrogène, et $R^2$ est le reste 4-méthylphényle.

6. Composé selon la revendication 4, dans lequel $R^1$ est le reste méthyle, et $R^2$ est le reste phényle.

7. Procédé de préparation des composés selon les revendications 1 à 6, qui comprend les étapes consistant à:

(A) faire réagir un composé de formule:

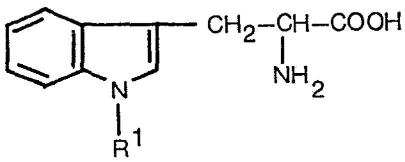

(II)

où $R^1$ a la même signification que ci-dessus, avec l'oxysulfure de carbone (COS) et un halogénure de benzyle de formule:

$$R^2CH_2X \quad (III)$$

où X est un halogène, et $R^2$ a la même signification que ci-dessus, ou bien (B) faire réagir le composé (II) avec un composé de formule:

$$R^2CH_2SCOX \quad (IV)$$

où $R^2$ et X ont la même signification que ci-dessus, et

(C) si nécessaire, convertir ensuite le produit en un sel de celui-ci pharmaceutiquement acceptable.

8. Procédé selon la revendication 7, dans lequel la réaction du composé (II) avec l'oxysulfure de carbone et le composé (III) est réalisée à une température comprise entre —30°C et 50°C, en présence d'un accepteur d'acide dans un solvant, ou bien la réaction du composé (II) avec le composé (IV) est réalisée à une température comprise entre —50°C et 50°C en présence d'un accepteur d'acide dans un solvant.

9. Composition pharmaceutique qui comprend un composé selon les revendications 1—6 et un véhicule pharmaceutique acceptable pour celui-ci.